# FASCICULE DE BREVET EUROPEEN

(11) **EP 2 833 928 B1**
(45) Date de publication et mention de la délivrance du brevet: **18.07.2018**
(21) Numéro de dépôt: 13712816.1
(22) Date de dépôt: 29.03.2013
(51) Int. Cl.: A61L 2/14, A61L 2/08

(54) **SYSTEME DE STERILISATION PAR BOMBARDEMENT D'ELECTRONS A ENCOMBREMENT REDUIT**
KOMPAKTES SYSTEM ZUR STERILISATION DURCH ELEKTRONENBESCHUSS
COMPACT SYSTEM FOR STERILIZATION BY BOMBARDMENT OF ELECTRONS

(30) Priorité: 02.04.2012 FR 1253006
(43) Date de publication de la demande: 11.02.2015
(73) Titulaire: Getinge La Calhene, 41100 Vendôme (FR)
(72) Inventeur: LIGER, Philippe, F-13530 Trets (FR); CLECH, Patrick, F-28700 Sainville (FR)
(74) Mandataire: Brevalex
(86) Numéro de dépôt international: PCT/EP2013/056821
(87) Numéro de publication internationale: WO 2013/149968

(56) Documents cités:
- EP-A1- 1 685 853
- EP-A1- 1 849 746
- WO-A2-2004/102587
- WO-A2-2006/111681

## Description

### DOMAINE TECHNIQUE ET ART ANTÉRIEUR

La présente invention se rapporte à un système de stérilisation par bombardement d'électrons à encombrement réduit.

La stérilisation par bombardement d'électrons est utilisée par exemple pour stériliser des objets destinés à être introduits dans un isolateur stérile utilisé dans la fabrication de produits pharmaceutiques.

Le système comporte un convoyeur destiné à transporter les objets à stériliser jusqu'à une zone de traitement où ils sont irradiés par un faisceau généré par un ou plusieurs accélérateurs d'électrons, puis jusqu'à la sortie du système, qui est par exemple directement reliée à l'isolateur.

Afin que le rayonnement ionisant généré par les accélérateurs d'électrons reste confiné dans la zone de traitement, des chicanes formées par des plaques de plomb verticales sont ménagées en amont et en aval de la zone de traitement. Le convoyeur qui se déplace dans un plan horizontal, est alors configuré pour contourner les plaques de plomb.

Le système offre un fonctionnement très satisfaisant et le confinement du rayonnement ionisant est assuré. Ce système présente cependant un encombrement au sol important. En effet, le parcours que les chicanes imposent au convoyeur est consommateur de place.

Le document EP1685853 décrit un système de stérilisation par bombardement d'électrons comportant une enceinte blindée munie d'une entrée et d'une sortie, d'une zone de traitement, d'une zone d'acheminement formée par le convoyeur d'entrée et d'une zone d'évacuation en aval de la zone de traitement formée par le convoyeur de sortie. Des parois de radioprotection sont prévues au niveau des zones d'acheminement et d'évacuation et des moyens de franchissement des parois de radioprotection permettant de passer au-dessus des parois de radioprotection.

### EXPOSÉ DE L'INVENTION

C'est par conséquent un but de la présente invention d'offrir un dispositif de stérilisation par bombardement d'électrons d'encombrement réduit.

Le but de la présente invention est atteint par un système de stérilisation par bombardement d'électrons comportant une zone de traitement, une zone d'acheminement en amont de la zone de traitement et une zone d'évacuation en aval de la zone de traitement, la zone d'acheminement et la zone d'évacuation comportant chacun une paroi de radioprotection, la zone d'acheminement et/ou la zone d'évacuation comportant un premier convoyeur en amont de la paroi de radioprotection et un deuxième convoyeur en aval de la paroi de radioprotection et des moyens pour déplacer un objet acheminé par le premier convoyeur vers le deuxième convoyeur pardessus la première paroi de radioprotection.

De préférence les zones d'acheminement et d'évacuation comportent chacune deux convoyeurs et des moyens pour faire franchir aux objets la paroi de radioprotection par au dessus.

En d'autres termes, le cheminement des objets à stériliser en amont et/ou en aval de la zone de traitement ne se fait plus uniquement dans un plan horizontal mais également dans un plan vertical, les convoyeurs amont et/ou aval sont remplacés par deux convoyeurs de forme simple circulant en amont et en aval de la paroi de radioprotection. L'encombrement des convoyeurs est réduit et leur réalisation est simplifiée. La longueur des zones amont et aval est de fait réduite ainsi que la dimension transversale.

De manière très avantageuse, les moyens pour déplacer l'objet d'un convoyeur à l'autre comportent un bras monté en rotation autour d'un axe horizontal et d'une pince. La pince saisit l'objet et le bras mis en rotation déplace l'objet d'un convoyeur à l'autre, la pince est ensuite écartée pour libérer l'objet.

Préférentiellement un automate commande les moyens de traitement des objets et le ou les dispositifs d'acheminement.

La présente invention a alors principalement pour objet un système de stérilisation d'objets par bombardement d'électrons comportant une enceinte blindée pour y confiner les rayonnements ionisants munie d'une entrée et d'une sortie par lesquelles entrent et sortent respectivement les objets, ledit système comportant entre l'entrée et la sortie, une zone de traitement, une zone d'acheminement en amont de la zone de traitement et une zone d'évacuation en aval de la zone de traitement, et des moyens de convoyage des objets de l'entrée à la sortie, la zone de traitement comportant au moins un accélérateur d'électrons et un convoyeur, la zone d'acheminement et la zone d'évacuation comportant chacune au moins une paroi de radioprotection contre les rayonnements ionisants et des convoyeur, et dans lequel la zone d'acheminement et/ou la zone d'évacuation comportent au moins un premier et deuxième convoyeur de part et d'autre de la paroi de radioprotection et un dispositif de franchissement de ladite paroi de radioprotection permettant aux objets de franchir la paroi de radioprotection en passant au dessus de ladite paroi de radioprotection.

De manière préférée, la zone d'acheminement et la zone d'évacuation comportent chacune au moins un premier et deuxième convoyeur de part et d'autre de la paroi de radioprotection et un dispositif de franchissement de ladite paroi de radioprotection permettant aux objets de franchir la paroi de radioprotection en passant au dessus de ladite paroi de radioprotection. Les moyens de franchissement comportent un bras monté par une première extrémité longitudinale sur un arbre de rotation monté en rotation autour d'un axe perpendiculaire à une direction de déplacement des objets, ledit axe étant contenu dans un plan situé entre le premier et le deuxième convoyeur, des moyens de préhension des objets montés sur une deuxième extrémité longitudinale du bras, des moyens de mise en rotation du bras de sorte qu'il effectue un mouvement de pivotement entre une extrémité du premier convoyeur et une extrémité du deuxième convoyeur et des moyens d'actionnement des moyens de préhension.

Les moyens de franchissement comportent avantageusement des moyens tels que les moyens de préhension se déplacent dans une succession de plans horizontaux. Lesdits moyens peuvent comporter une première poulie fixe, dans laquelle l'arbre de rotation tourne librement, une deuxième poulie montée libre en rotation sur la deuxième extrémité longitudinale du bras, une courroie reliant les première et deuxième poulies. Les moyens de préhension sont solidaires en rotation de la deuxième poulie.

Les moyens de préhension comportent par exemple un support, deux mâchoires, lesdites mâchoires étant articulées en rotation sur le support, chacune autour d'un axe parallèle à la direction de déplacement, des moyens de rappel élastique ramenant les mâchoires l'une vers l'autre.

Avantageusement, les moyens d'actionnement ne sont pas portés par le bras.

Par exemple, les moyens d'actionnement comportent une partie d'actionnement disposée au niveau de l'extrémité du premier convoyeur et une partie d'actionnement disposée au niveau de l'extrémité du deuxième convoyeur. Chaque partie d'actionnement peut comporter un arbre monté en rotation perpendiculairement à la direction de déplacement, ledit arbre étant muni de deux cames, chaque came étant destinée à coopérer avec chacune des mâchoires pour provoquer le pivotement desdites mâchoires autour de leur axe de rotation et des moyens de rotation des arbres d'un angle donné.

Dans un exemple, chaque mâchoire comporte deux doigts situés à chacune des extrémités de ladite mâchoire, lesdits doigts étant d'axe parallèle et distinct de l'axe de rotation de la mâchoire qui le porte, chaque came entrant en contact avec un doigt par rotation de l'arbre.

Les moyens de rotation des arbres d'un angle donné sont avantageusement formés par des vérins pneumatiques rotatifs connectés aux arbres par des systèmes de transmission à bielle.

Les objets peuvent comporter deux nervures et les mâchoires comportent chacune une rainure pour recevoir une nervure. Les objets sont par exemple des boîtes à section rectangulaire.

Le système de stérilisation comporte de préférence des moyens de détection de la position des objets à l'intérieur de chacune des zones. Le système de stérilisation comporte avantageusement un automate contrôlant le ou les dispositifs de franchissement et le traitement des objets par irradiation. Les convoyeurs sont par exemple des tapis à mailles ou des rouleaux.

### BRÈVE DESCRIPTION DES DESSINS

La présente invention sera mieux comprise à l'aide de la description qui va suivre et des dessins en annexe sur lesquels :
- la figure 1 est une représentation schématique illustrant le principe de fonctionnement d'un système de stérilisation selon l'invention,
- la figure 2 est une vue en coupe d'un exemple de réalisation d'un système de stérilisation,
- la figure 3 est une vue en perspective de la zone amont ou aval du système de stérilisation de la figure 2, dans différents états de manipulation des objets,
- la figure 4 est une vue agrandie d'une partie du système de la figure 3,
- la figure 5 est une vue de détail d'une partie d'actionnement des moyens de préhension du dispositif de franchissement,
- la figure 6 est une représentation schématique d'un exemple de trajectoire suivie par un objet lorsqu'il franchit une paroi de radioprotection dans uns système de stérilisation selon la présente invention.

### EXPOSÉ DÉTAILLÉ DE MODES DE RÉALISATION PARTICULIERS

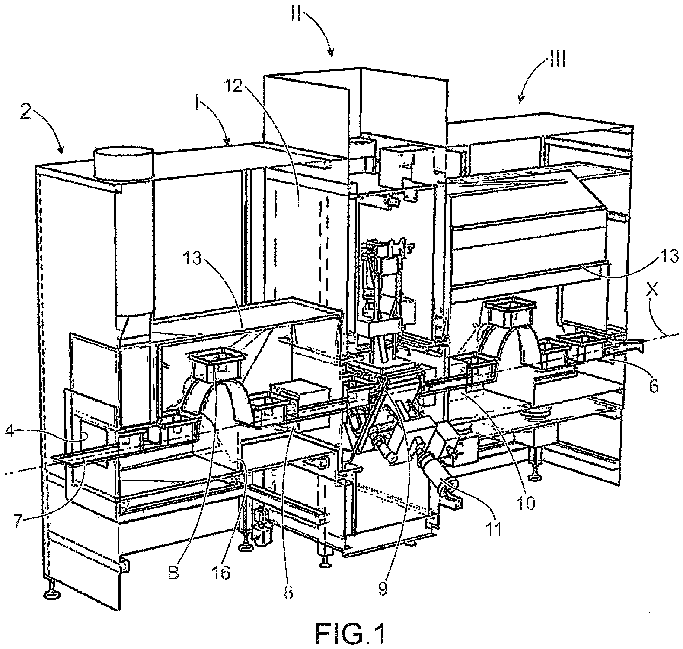

Sur la figure 1, on peut voir une représentation schématique de l'intérieur d'un système de stérilisation selon un exemple de réalisation illustrant le principe de l'invention.

Le système comporte une enveloppe 2 munie d'une entrée 4 et d'une sortie 6. A l'intérieur de l'enceinte 2, des objets se déplacent selon une direction X de l'entrée 4 vers la sortie 6.

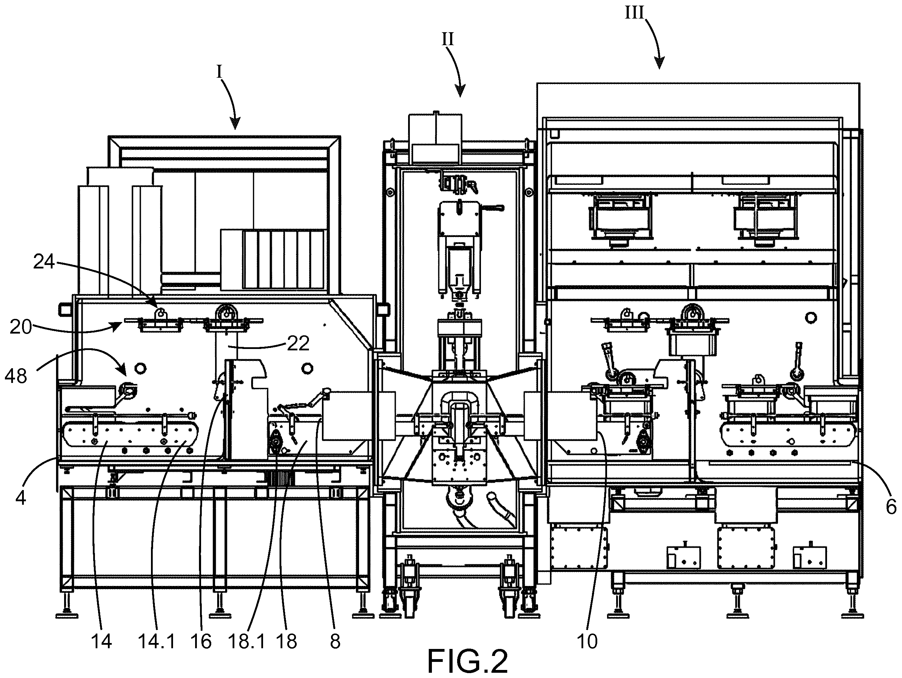

Dans la suite de la description, l'amont et l'aval sont considérés de l'entrée vers la sortie (de la gauche vers la droite sur la représentation des figures 1 et 2).

Par exemple la sortie 6 est connectée de à un isolateur (non représenté).

Le système comporte disposé d'amont en aval trois zones principales :
- une zone I, dite zone d'acheminement,
- une zone II, dite zone de traitement,
- une zone III, dite zone d'évacuation.

La zone d'acheminement I s'étend de l'entrée 4 à un passage d'entrée 8 de la zone de traitement II, et la zone d'évacuation III s'étend d'un passage de sortie 10 de la zone de traitement II vers la sortie 6.

Le système de stérilisation comporte un ensemble des barrières de radioprotection qui permet de garder les rayonnements ionisants à l'intérieur de l'enveloppe 2 entre l'entrée 4 et la sortie 6. Cet ensemble est formé de tunnels 13 dans les zones I et III, d'une enceinte qui entoure le ou les accélérateurs et le tunnel d'irradiation, enceinte qui est raccordée aux tunnels 13 par les passages d'entrée 8 et de sortie 10 de la zone de traitement II, et des parois verticales de radioprotection 16 dans les tunnels 13 des zones I et III.

La zone de traitement II comporte un convoyeur 9 et un ou plusieurs accélérateurs d'électrons 11 destinés à bombarder d'électrons les objets traversant le système en vue de stériliser leur surface. Dans l'exemple représenté, la zone de traitement II comporte trois accélérateurs dont deux seulement sont visibles, orientés à 120° les uns par rapport aux autres autour de la direction X. La zone de traitement II est séparée des zones d'acheminement I et zone d'évacuation III par deux parois 12 s'étendant perpendiculairement à la direction X et percée du passage d'entrée 8 et du passage de sortie 10.

La zone d'acheminement I et la zone d'évacuation III sont de structures semblables, seule la zone d'acheminement I sera décrite en détail à l'aide des figures 2 à 6.

La zone d'acheminement I est délimitée par le tunnel 13 déjà décrit formant un blindage de confinement des rayonnements ionisants pour la radioprotection. La zone d'acheminement I comporte d'amont en aval un premier convoyeur 14 s'étendant selon la direction X à partir de l'entrée 4, une paroi de radioprotection 16 formant un blindage pour confiner le rayonnement ionisant du côté de la zone de traitement II et un deuxième convoyeur 18 s'étendant selon la direction X jusqu'au passage d'entrée 8 de la zone de traitement II.

Les convoyeurs 14, 18 sont par exemple des tapis à mailles entraînés par engrenage ou des rouleaux qui peuvent être motorisés.

La paroi de radioprotection 16 est généralement en plomb. La paroi de radioprotection 16 s'étend perpendiculairement à la direction X et est en regard du passage d'entrée 8.

La zone d'acheminement I comporte également des moyens permettant aux objets à traiter de franchir la paroi de radioprotection pour passer entre le premier convoyeur 14 et le deuxième convoyeur 18.

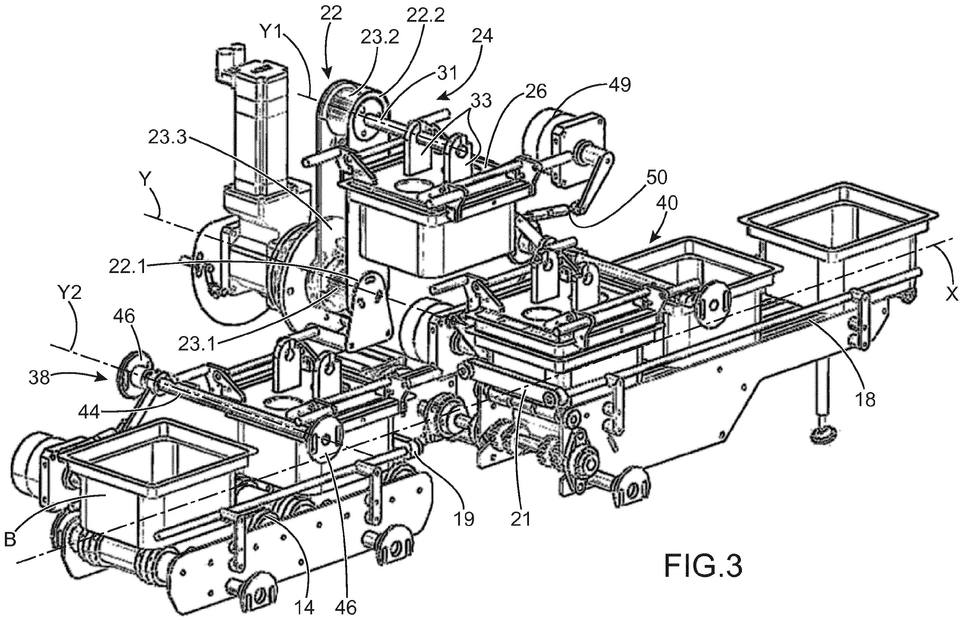

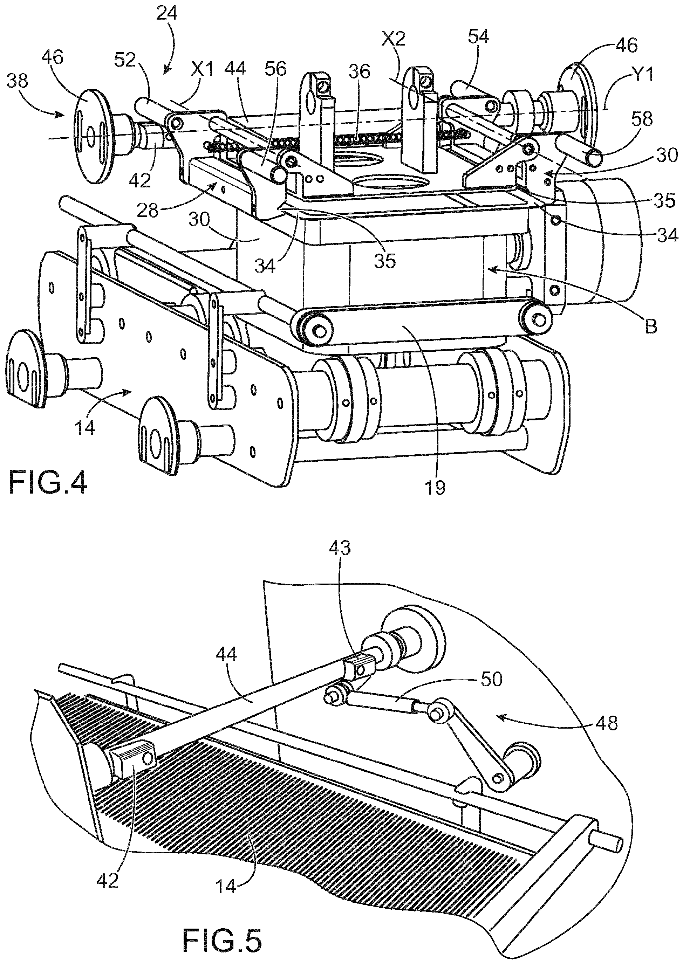

De préférence, une butée 19 est prévue au niveau de l'extrémité aval du premier convoyeur 14 avant la paroi de radioprotection 16 pour arrêter l'objet (figure 4). De même à l'entrée du deuxième convoyeur 18, une butée 21 est également prévue (figure 3). Celle-ci peut par exemple servir lorsque les convoyeurs 14, 18 cheminent en sens inverse, l'entrée du deuxième convoyeur 18 formant alors la sortie du deuxième convoyeur.

Sur la figure 1, les objets sont des boîtes B et leur trajectoire dans le système est représentée par une flèche désignée T.

On observe que les boîtes B se déplacent dans un plan horizontal en amont de la paroi 16 grâce au premier convoyeur 14, puis se déplacent à la fois selon une direction verticale et selon une direction horizontale pour franchir la paroi 16, puis se déplacent à nouveau dans un plan horizontal en aval de la paroi 16 grâce au deuxième convoyeur 18.

Grâce à se franchissement de la paroi par le dessus de celle-ci, la longueur de la zone d'acheminement est réduite. Il en va de même pour la longueur de la zone d'évacuation III.

La dimension transversale des zones d'acheminement et d'évacuation est également réduite par rapport aux systèmes de l'état de la technique car il n'y plus de convoyeur cheminant horizontalement pour contourner les parois.

Nous allons maintenant décrire en détail un exemple de réalisation particulièrement avantageux d'un dispositif de franchissement 20 particulièrement visible sur les figures 3, 4 et 5.

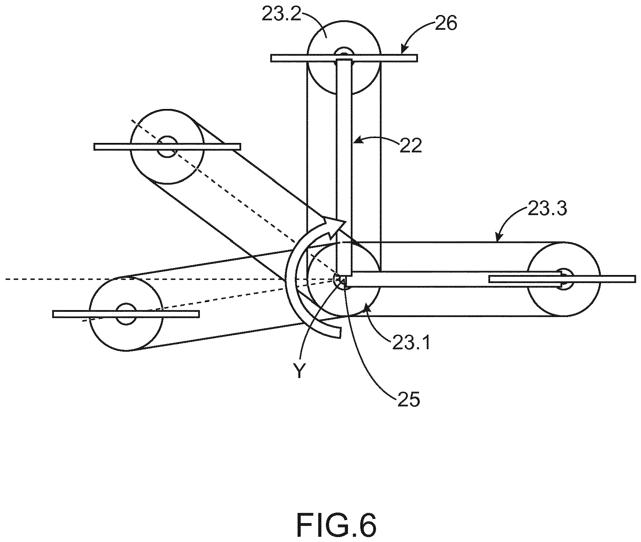

Le dispositif de franchissement 20 est situé entre le premier convoyeur 14 et le deuxième convoyeur 18. Il comporte un bras 22 monté sur l'extrémité d'un arbre de rotation 25 (figure 6) mobile en rotation autour s'étendant selon un axe Y perpendiculaire à la direction X et situé dans un plan s'étendant une extrémité de sortie 14.1 du premier convoyeur et une extrémité d'entrée 18.1 du deuxième convoyeur 18. Le bras 22 est articulé autour de l'axe Y par une première extrémité 22.1 et porte à sa deuxième extrémité 22.2 des moyens de préhension 24 destinés à venir saisir les objets B à traiter.

Les moyens d'entraînement par exemple formés par un moteur en prise directe avec l'arbre de rotation 25.

De manière avantageuse, le bras 22 comporte des moyens pour maintenir les moyens de préhension horizontaux, et donc la boîte B, pendant le franchissement.

Ces moyens comportent, dans l'exemple représenté, une première poulie 23.1 montée fixe par rapport à l'ensemble du système : le tunnel 13, les parois de radioprotection. L'arbre de rotation 25 passe à travers la première poulie 23.1 et est libre de rotation par rapport à la poulie 23.1. Les moyens comportent une deuxième poulie 23.2 montée libre en rotation autour de l'axe Y1, une courroie 23.3 reliant les deux poulies. Les moyens de préhension comportent un support 26 solidaire de la poulie 23.2. Ainsi lors du déplacement du bras 22, le support 26 conserve sa position horizontale.

Sur la figure 6, on peut voir le bras dans différentes positions lors de son déplacement entre le premier 14 et le deuxième 18 convoyeur. La boîte B a alors une trajectoire en forme d'arc de cercle entre les deux convoyeurs. La réalisation du bras 22 et son entraînement présentent l'avantage d'être très simples.

En variante on pourrait prévoir un système tel que la trajectoire de la boîte soit parabolique, voire plus complexe. La distance entre les deux convoyeurs serait alors plus petite que la hauteur à laquelle la boîte doit être amenée pour franchir la cloison 16. La longueur du système serait alors encore réduite.

Dans l'exemple représenté, les moyens de préhension 24 sont adaptés à la préhension de boîtes de forme rectangulaire.

Nous allons décrire la forme des boîtes qui sont manipulées par les moyens de préhension 24 dans l'exemple représenté. Les boîtes, particulièrement visibles sur la figure 4, sont de forme rectangulaire. La boîte B est disposée sur les convoyeurs 14, 18 de sorte que ses parois latérales 30 soient parallèles à la direction X. Le bord supérieur de chaque paroi latérale 30 comporte une nervure 34 s'étendant perpendiculaire au plan de la paroi latérale vers l'extérieur de la boîte et sur une partie au moins de la longueur de la paroi latérale 30.

Les moyens de préhension 24 comportent le support 26 précédemment décrit, sur lequel sont articulées deux mâchoires 28, 30 autour d'axes X1, X2 respectivement. Les axes X1 et X2 sont parallèles à la direction X. Chaque mâchoire 28, 30 comporte une rainure 35 à profil en V dont l'axe est parallèle à la direction X et est apte à recevoir une nervure 34 du bord supérieur d'une paroi latérale 30 de la boîte B. Le support 26 est solidaire en rotation de la poulie 23.2. Dans l'exemple représenté, un axe 31 solidaire de la poulie s'étendant suivant l'axe Y1 est monté dans deux étriers 33 fixés sur la face supérieure du support 26 (figure 3).

L'horizontalité du support 26 et donc de la boîte transportée est assuré.

Des moyens élastiques de rappel 36 sont prévus entre les deux mâchoires 28, 30 pour les rappeler automatiquement l'une vers l'autre en position de serrage. Dans l'exemple représenté, les moyens élastiques de rappel sont formés par un ressort hélicoïdal fixé par chacune de ses extrémités à une mâchoire 28, 30.

Dans l'exemple représenté et de manière très avantageuse, les moyens de préhension 24 sont actionnés par des moyens extérieurs qui ne sont pas embarqués sur le bras, ce qui permet de réduire la charge déplacée par le bras 22. Néanmoins, on pourrait envisager que des moyens d'actionnement par exemple électriques des moyens de préhension soient embarqués sur le bras, par exemple montés sur le support.

Nous allons maintenant décrire les moyens d'actionnement des moyens de préhension.

Les moyens de préhension sont actionnés une première fois pour saisir la boîte B lorsque celle-ci se situe à la sortie 16.1 du premier convoyeur 14 et une deuxième fois pour libérer la boîte B lorsque celle-ci a franchi la cloison 16 et se trouve à l'entrée 18.1 du deuxième convoyeur 18.

Dans l'exemple représenté et de manière très avantageuse, les moyens d'actionnement comportent une partie d'actionnement amont 38 pour assurer la saisie de la boîte et une partie d'actionnement aval 40 pour libérer la boîte B dans le mode de déplacement de l'amont vers l'aval. Nous verrons dans la suite de la description que le mode de déplacement peut être inversé.

Les deux parties d'actionnement amont et aval 38,40 sont de structure similaire, seule la partie d'actionnement amont 38 sera décrite en détail.

La partie d'actionnement amont 38 comporte deux cames 42, 43 portées par un arbre 44 disposé perpendiculairement à la direction X et disposé au niveau de la sortie du premier convoyeur 14. L'arbre 44 est monté mobile en rotation autour d'un axe Y2. Dans l'exemple représenté, les extrémités longitudinales de l'arbre 44 sont montées dans deux paliers 46 fixés sur les parois latérales de l'enceinte 13.

Des moyens de mise en rotation 48 de l'arbre 44 autour de l'axe Y2 sont prévus, ceux-ci sont particulièrement visibles sur la figure 5. Dans l'exemple représenté, les moyens 48 sont déportés et comportent un vérin pneumatique 49 rotatif (figure 3) qui entraine l'arbre 44 via un système de transmission à biellette 50.

De tels moyens de mise en rotation 48 présentent un certains nombres d'avantages.

D'une part, on peut prévoir que la longueur de la biellette 50 soit ajustable pour permettre l'ajustement de la course de rotation de l'arbre 44 en fonction de la course du vérin pneumatique.

D'autre part, en réalisant préférentiellement la biellette 50 au moins en partie un matériau apte à se rompre en cas de contrainte mécanique trop élevée, par exemple en matériau plastique, on protège le reste de la structure. En effet, c'est la biellette qui se casse préférentiellement par rapport au reste du système. Elle joue donc avantageusement le rôle de fusible mécanique.

Enfin, cette réalisation permet le montage/démontage de l'arbre 44 ou du vérin indépendamment l'un de l'autre

En variante, on pourrait prévoir de monter un moteur électrique directement en bout d'arbre 44, ou de remplacer le vérin pneumatique rotatif par un moteur électrique.

Dans l'exemple représenté, l'arbre 44 pivote d'un quart de tour dans un sens de rotation et dans le sens de rotation inverse.

Chaque came 42, 43 de l'arbre 44 est destinée à coopérer avec un doigt 52, 54 portés par la mâchoire 28, 30 respectivement au niveau de l'extrémité longitudinale amont de la mâchoire, les doigts 52, 54 présentent des axes longitudinaux parallèles aux axes X1, X2 et distincts des axes X1, X2. Ainsi un déplacement vers le haut du doigt 52, 54 provoque une rotation de la mâchoire 28, 30 sur laquelle il est fixé, autour de son axe X1, X2.

Les mâchoires 28, 30 comportent également des doigts 56, 58 au niveau de leur extrémité longitudinale aval destines à coopérer avec la partie d'actionnement 40 en entrée du deuxième convoyeur 18.

Dans un autre exemple de réalisation, on pourrait envisager de supprimer les doigts.

Les moyens de préhension et d'actionnement présentent l'avantage d'être entièrement réversibles, ce qui permet de pouvoir convoyer les objets en sens inverse, par exemple en cas de bourrage ou de coincement d'un ou de plusieurs objets dans le système.

De manière avantageuse, le processus de convoyage et le processus d'irradiation sont contrôlés par un automate qui reçoit les signaux de détection de présence de l'objet émis par des détecteurs disposés à différents endroits sur le parcours des convoyeurs, les paramètres de fonctionnement des accélérateurs et les informations des machines qui sont interfacées avec le système de stérilisation, en particulier l'isolateur situé en sortie du système de stérilisation.

L'acheminement d'une boîte B de l'entrée 4 du système à la sortie 6 va maintenant être décrit.

Sur la figure 3, les moyens de préhension 24 sont représentés dans un état de préhension de la boîte sur le convoyeur 14, dans un état de franchissement au dessus de la paroi de radioprotection 16 et dans un état de libération de la boîte sur le convoyeur 18.

A l'état initial, le bras 22 est sensiblement à la verticale (figure 2).

La boîte B est disposée à l'entrée 4 de la zone d'acheminement I sur l'extrémité aval du premier convoyeur 14. Elle est alors acheminée jusqu'à l'extrémité 14.1 du premier convoyeur 14. La présence de la boîte B est alors détectée.

Le bras rotatif 22 est mis en rotation dans le sens antihoraire sur la figure 2 pour positionner les moyens de préhension au dessus de la boîte B et positionner les doigts 52, 54 des mâchoires 28, 30 à l'aplomb des cames 42, 43. L'arbre 44 est alors mis en rotation d'un quart de tour par le système de transmission à bielle 50, ce qui provoque le basculement des mâchoires 28, 30 autour des axes X1, X2. Les mâchoires s'écartent, permettant à la partie supérieure de la boîte B de se positionner entre les mâchoires 28, 30.

Le système de transmission à bielle 50 est à nouveau activé provoquant à nouveau une rotation d'un quart de tour du bras 44. Les cames 46 ne sont plus en contact avec les doigts 52, 54. Sous l'action du ressort de rappel 36, les mâchoires 28, 30 se rapprochent l'une de l'autre, les nervures 34 de la boîte B sont alors reçues dans les rainures 35 des mâchoires 28, 30, la boîte B est alors solidaire des mâchoires 28, 30.

Le bras 22 est ensuite pivoté dans le sens horaire pour faire franchir à la boîte B la paroi de radioprotection 16 et la positionner à l'entrée 18.1 du deuxième convoyeur 18.

Les doigts 56, 58 se retrouvent alors à l'aplomb des cames de la partie d'actionnement aval. Le système de transmission à bielle aval est alors à son tour activé, les cames pivotent entrent en contact avec les doigts 56, 58, ce qui a pour effet d'écarter les mâchoires 28, 30. La boîte B est alors libérée et la boîte B peut alors être emportée par le deuxième convoyeur 18 jusqu'à atteindre la zone de traitement II dans laquelle sa ou ses surfaces extérieures sont stérilisées par un bombardement d'électrons.

La boîte B sort ensuite de la zone de traitement II, pénètre dans la zone d'évacuation III. La boîte est alors acheminée à travers la zone d'évacuation III de manière similaire à celle dans la zone d'acheminement I : déplacement sur un premier convoyeur, franchissement d'une paroi de radioprotection par le dispositif de franchissement similaire au dispositif de franchissement 20, puis est transportée jusqu'à la sortie 6 du système. Sur la figure 2, on peut voir la boîte B en train de franchir la paroi de radioprotection 16 de la zone d'évacuation III.

Par exemple, lorsqu'un système de stérilisation de l'état de la technique a une longueur de 4500 mm, le système de stérilisation selon l'invention a une longueur de 4000 mm, ce qui représente une réduction de longueur substantielle. La largeur du système est également sensiblement réduite, un système de stérilisation de l'état de la technique a une largeur de 4467 mm, le système de stérilisation selon l'invention peut présenter une largeur de 3964 mm,

A titre d'exemple, le système de stérilisation peut être programmé pour fournir en sortie 6, six boîtes B traitées par minute. Le temps de franchissement des parois de radioprotections peut-être de l'ordre de 3 s et la vitesse de convoyage dans les zones I et III est de l'ordre de 5,4 m/min et la dans la zone II de 2 m/min.

Le système de stérilisation présente donc également l'avantage de pouvoir tenir une cadence élevée de fourniture d'objets stérilisés.

Le système et en particulier les moyens de préhension ont été décrits pour la stérilisation de boîtes rectangulaires, plus généralement d'objets rectangulaires. Il sera compris que le système est adapté à la stérilisation d'objets de forme variée, comme par exemple des boîtes de forme circulaire, les mâchoires étant alors formées de deux arcs de cercle.

Le dispositif de franchissement décrit en détail est particulièrement avantageux du fait de sa simplicité et de sa robustesse, mais également de sa rapidité d'action. Néanmoins d'autres dispositifs de franchissement pourraient être utilisés, tels qu'un dispositif à grappin, un dispositif magnétique ou un dispositif à ventouse pneumatique si le matériau et la forme des objets sont adaptés...

En outre, dans l'exemple décrit les convoyeurs amont et aval sont contenus dans un même plan horizontal. Un système dans lequel les convoyeurs amont et aval seraient dans deux plans horizontaux distincts ou dans lequel les convoyeurs seraient dans des plans inclinés ne sort pas du cadre de la présente invention.

Par ailleurs, un système dans lequel par exemple uniquement la zone de cheminement ou la zone d'évacuation comporterait deux convoyeurs et un dispositif de franchissement et l'autre zone comporterait un seul convoyeur contournant la paroi de radioprotection sur le côté ne sort pas du cadre de la présente invention. Un tel système présente également un encombrement réduit par rapport aux systèmes de l'état de la technique.

On pourrait envisager que la zone d'acheminement et/ou la zone d'évacuation comportent chacune plusieurs parois de protection, les zones comporterait alors autant de dispositifs de franchissement que de parois.

## Revendications

1. Système de stérilisation d'objets (B) par bombardement d'électrons comportant une enceinte blindée pour y confiner les rayonnements ionisants (2) munie d'une entrée (4) et d'une sortie (6) par lesquelles entrent et sortent respectivement les objets (B), ledit système comportant entre l'entrée (4) et la sortie (6), une zone de traitement (II), une zone d'acheminement (I) en amont de la zone de traitement (II) et une zone d'évacuation (III) en aval de la zone de traitement (II), et des moyens de convoyage (9, 14, 18) des objets (B) de l'entrée (4) à la sortie (6), la zone de traitement (II) comportant au moins un accélérateur d'électrons et un convoyeur (9), la zone d'acheminement (1) et la zone d'évacuation (III) comportant chacune au moins une paroi de radioprotection (16) contre les rayonnements ionisants et des convoyeurs (14, 18), la zone d'acheminement (1) et/ou la zone d'évacuation (II) comportant au moins un premier (14) et deuxième (18) convoyeur de part et d'autre de la paroi de radioprotection (16) et un dispositif de franchissement (20) de ladite paroi de radioprotection (16) permettant aux objets (B) de franchir la paroi de radioprotection (16) en passant au-dessus de ladite paroi de radioprotection (16), **caractérisé en ce que** les moyens de franchissement (20) comportent un bras (22) monté par une première extrémité longitudinale (22.1) sur un arbre de rotation (25) monté en rotation autour d'un axe (Y) perpendiculaire à une direction de déplacement (X) des objets (B), ledit axe (Y) étant contenu dans un plan situé entre le premier (14) et le deuxième (18) convoyeur, des moyens de préhension (24) des objets montés sur une deuxième extrémité longitudinale (22.2) du bras (22), des moyens de mise en rotation du bras de sorte qu'il effectue un mouvement de pivotement entre une extrémité (14.1) du premier convoyeur (14) et une extrémité (18.1) du deuxième convoyeur (18) et des moyens d'actionnement des moyens de préhension (24).

2. Système de stérilisation selon la revendication 1, dans lequel la zone d'acheminement (I) et la zone d'évacuation (II) comportent chacune au moins un premier (14) et deuxième (18) convoyeur de part et d'autre de la paroi de radioprotection (16) et un dispositif de franchissement (20) de ladite paroi de radioprotection (16) permettant aux objets (B) de franchir la paroi de radioprotection (16) en passant au-dessus de ladite paroi de radioprotection (16).

3. Système de stérilisation selon la revendication 1 ou 2, dans lequel les moyens de franchissement comportent des moyens tels que les moyens de préhension se déplacent dans une succession de plans horizontaux.

4. Système de stérilisation selon la revendication 3, dans lequel lesdits moyens comportent une première poulie (23.1) fixe, dans laquelle l'arbre de rotation (25) tourne librement, une deuxième poulie (23.2) montée libre en rotation sur la deuxième extrémité longitudinale (22.2) du bras (22), une courroie (23.3) reliant les première (23.1) et deuxième (23.2) poulies et dans lequel les moyens de préhension (24) sont solidaires en rotation de la deuxième poulie (23.2).

5. Système de stérilisation selon l'une des revendications 1 à 4, dans lequel les moyens de préhension (24) comportent un support (26), deux mâchoires (28, 30), lesdites mâchoires (28, 30) étant articulées en rotation sur le support (26), chacune autour d'un axe (X1, X2) parallèle à la direction de déplacement (X), des moyens de rappel élastique (36) ramenant les mâchoires (28, 30) l'une vers l'autre.

6. Système de stérilisation selon l'une des revendications 1 à 5, dans lequel les moyens d'actionnement ne sont pas portés par le bras (22).

7. Système de stérilisation selon la revendication 6, dans lequel les moyens d'actionnement comportent une partie d'actionnement (38) disposée au niveau de l'extrémité (14.1) du premier convoyeur (14) et une partie d'actionnement (40) disposée au niveau de l'extrémité (18.1) du deuxième convoyeur (18).

8. Système de stérilisation selon la revendication 7, dans lequel chaque partie d'actionnement comporte un arbre (44) monté en rotation perpendiculairement à la direction de déplacement (X), ledit arbre (44) étant muni de deux cames (42, 43), chaque came (42, 43) étant destinée à coopérer avec chacune des mâchoires (28, 30) pour provoquer le pivotement desdites mâchoires (28, 30) autour de leur axe de rotation (X1,X2) et des moyens de rotation des arbres (44) d'un angle donné.

9. Système de stérilisation selon la revendication 8, dans lequel chaque mâchoire (28, 30) comporte deux doigts (52, 54, 56, 58) situés à chacune des extrémités de ladite mâchoire (28, 30), lesdits doigts (52, 54, 56, 58) étant d'axe parallèle et distinct de l'axe de rotation de la mâchoire (28, 30) qui le porte, chaque came (42, 43) entrant en contact avec un doigt (52, 54, 56, 58) par rotation de l'arbre (44).

10. Système de stérilisation selon la revendication 8 ou 9, dans lequel les moyens (50) de rotation des arbres (44) d'un angle donné sont formés par des vérins pneumatiques rotatifs connectés aux arbres (44) par des systèmes de transmission à bielle (50).

11. Système de stérilisation selon l'une des revendications 1 à 10 pris en combinaison avec la revendication 5, dans lequel les objets (B) comportent deux nervures (34) et les mâchoires (28, 30) comportent chacune une rainure (35) pour recevoir une nervure (34).

12. Système de stérilisation selon l'une des revendications 1 à 11, comportant des moyens de détection de la position des objets à l'intérieur de chacune des zones.

13. Système de stérilisation selon l'une des revendications 1 à 12, comportant un automate contrôlant le ou les dispositifs de franchissement (20) et le traitement des objets par irradiation.

14. Système de stérilisation selon l'une des revendications 1 à 13, dans lequel les convoyeurs (9, 14, 18) sont des tapis à mailles ou des rouleaux.

## Patentansprüche

1. Sterilisationssystem zur Sterilisation von Objekten (B) durch Elektronenbeschuss, aufweisend ein gepanzertes Gehäuse (2) zum Zurückhalten der ionisierenden Strahlen darin, das mit einem Eingang (4) und einem Ausgang (6) versehen ist, über welche die Objekte (B) eintreten bzw. austreten, wobei
das System zwischen dem Eingang (4) und dem Ausgang (6) eine Behandlungszone (II), eine Einlaufzone (I) vor der Behandlungszone (II) und eine Auslaufzone (III) nach der Behandlungszone (II) sowie Fördereinrichtungen (9, 14, 18) zum Befördern der Objekte (B) von dem Eingang (4) zu dem Ausgang (6) aufweist,
die Behandlungszone (II) mindestens einen Elektronenbeschleuniger und eine Fördereinrichtung (9) aufweist,
sowohl die Einlaufzone (I) als auch die Auslaufzone (III) mindestens eine Strahlenschutzwand (16) gegen ionisierende Strahlen und Fördereinrichtungen (14, 18) aufweist,
die Einlaufzone (I) und/oder die Auslaufzone (II) mindestens eine erste (14) und zweite (18) Fördereinrichtung beiderseits der Strahlenschutzwand (16) sowie eine Passiervorrichtung (20) zum Passieren der Strahlenschutzwand (16) aufweist, um den Objekten (B) ein Passieren der Strahlenschutzwand (16) zu erlauben, indem sie die Strahlenschutzwand (16) oberhalb überqueren,
**dadurch gekennzeichnet, dass** die Passiereinrichtungen (20) aufweisen:
einen Arm (22), der mit einem ersten Längsende (22.1) an einer Drehwelle (25) angebracht ist, die sich um eine Achse (Y) dreht, die senkrecht zu einer Verschiebungsrichtung (X) der Objekte (B) ist, wobei die Achse (Y) in einer zwischen der ersten (14) und zweiten (18) Fördereinrichtung angeordneten Ebene enthalten ist,
an einem zweiten Längsende (22.2) des Arms (22) angebrachte Greifeinrichtungen (24) zum Greifen der Objekte,
Einrichtungen zum Drehen des Arms, so dass er eine Schwenkbewegung zwischen einem Ende (14.1) der ersten Fördereinrichtung (14) und einem Ende (18.1) der zweiten Fördereinrichtung (18) ausführt, und
Einrichtungen zum Betätigen der Greifeinrichtungen (24).

2. Sterilisationssystem nach Anspruch 1, in welchem die Einlaufzone (I) und/oder die Auslaufzone (II) mindestens eine erste (14) und zweite (18) Fördereinrichtung beiderseits der Strahlenschutzwand (16) sowie eine Passiervorrichtung (20) zum Passieren der Strahlenschutzwand (16) aufweist, um den Objekten (B) ein Passieren der Strahlenschutzwand (16) zu erlauben, indem sie die Strahlenschutzwand (16) oberhalb überqueren.

3. Sterilisationssystem nach Anspruch 1 oder 2, in welchem die Passiereinrichtungen solche Mittel aufweisen, dass die Greifeinrichtungen sich in einer Folge von horizontalen Ebenen verschieben.

4. Sterilisationssystem nach Anspruch 3, in welchem die Mittel eine erste feststehende Scheibe (23.1), in der die Drehwelle (25) sich frei dreht, eine zweite Scheibe (23.2), die frei drehend an dem zweiten Längsende (22.2) des Arms (22) angebracht ist, und einen die erste (23.1) und zweite (23.2) Scheibe verbindenden Treibriemen (23.3) aufweisen, und in welchem die Greifeinrichtungen (24) sich mit der zweiten Scheibe (23.2) drehen.

5. Sterilisationssystem nach einem der Ansprüche 1 bis 4, in welchem die Greifeinrichtungen (24) aufweisen:
einen Träger (26),
zwei Klemmbacken (28, 30), wobei die Klemmbacken (28, 30) gelenkig an dem Träger (26) angebracht sind und jeweils um eine zur Verschiebungsrichtung (X) parallele Achse (X1, X2) drehbar sind,
elastische Rückstelleinrichtungen (36), die die Klemmbacken (28, 30) zueinander hin zurückstellen.

6. Sterilisationssystem nach einem der Ansprüche 1 bis 5, in welchem die Betätigungseinrichtungen nicht von dem Arm (22) getragen werden.

7. Sterilisationssystem nach Anspruch 6, in welchem die Betätigungseinrichtungen einen im Bereich des Endes (14.1) der ersten Fördereinrichtung (14) angeordneten Betätigungsteil (38) und einen im Bereich des Endes (18.1) der zweiten Fördereinrichtung (18) angeordneten Betätigungsteil (40) aufweisen.

8. Sterilisationssystem nach Anspruch 7, in welchem jeder Betätigungsteil aufweist:
eine sich senkrecht zur Verschiebungsrichtung (X) erstreckende drehende Welle (44), wobei die Welle (44) mit zwei Nocken (42, 43) versehen ist, wobei jeder Nocken (42, 43) konfiguriert ist, um mit der jeweiligen Klemmbacke (28, 30) zusammenzuarbeiten, um die Drehung der Klemmbacken (28, 30) um ihre Drehachse (X1, X2) zu bewirken, und
Einrichtungen zum Drehen der Wellen (44) um einen gegebenen Winkel.

9. Sterilisationssystem nach Anspruch 8, in welchem jede Klemmbacke (28, 30) zwei Finger (52, 54, 56, 58) aufweist, die jeweils an Enden der Klemmbacke (28, 30) angeordnet sind, wobei die Finger (52, 54, 56, 58) sich parallel zur und in Abstand von der Drehachse der jeweiligen Klemmbacke (28, 30) erstrecken, wobei jeder Nocken (43, 43) durch Drehung der Welle (44) in Kontakt mit einem Finger (52, 54, 56, 58) kommt.

10. Sterilisationssystem nach Anspruch 8 oder 9, in welchem die Einrichtungen (50) zum Drehen der Wellen (44) um einen gegebenen Winkel aus pneumatischen Schraubenspindeln gebildet sind, die über Pleuelstangen-Kraftübertragungssysteme (50) mit den Wellen (44) verbunden sind.

11. Sterilisationssystem nach einem der Ansprüche 1 bis 10 in Kombination mit Anspruch 5, in welchem die Objekte (B) zwei Rippen (34) aufweisen und die Klemmbacken (28, 30) jeweils eine Rille (35) zur Aufnahme einer Rippe (34) aufweisen.

12. Sterilisationssystem nach einem der Ansprüche 1 bis 11, aufweisend Einrichtungen zum Detektieren der Position der Objekte im Innern jeder Zone.

13. Sterilisationssystem nach einem der Ansprüche 1 bis 12, aufweisend einen Automat, der die Passiervorrichtung bzw. die Passiervorrichtungen (20) und die Strahlenbehandlung der Objekte steuert.

14. Sterilisationssystem nach einem der Ansprüche 1 bis 13, in welchem die Fördereinrichtungen (9, 14, 18) Gliederbänder oder Rollen sind.

## Claims

1. System for sterilization of objects (B) by electron bombardment comprising a shielded chamber to confine ionising radiation (2) provided with an inlet (4) and an outlet (6) through which objects (B) are input and output respectively, said system between the inlet (4) and the outlet (6) comprising a treatment zone (II), a routing zone (I) upstream from the treatment zone (II) and an evacuation zone (III) downstream from the treatment zone (II), and means (9, 14, 18) of conveying objects (B) from the inlet (4) to the outlet (6), the treatment zone (II) comprising at least one electron accelerator and a conveyor (9), the routing zone (I) and the evacuation zone (III) each including at least one radiation shielding wall (16) protecting against ionising radiation and conveyors (14, 18), the routing zone (I) and/or the evacuation zone (II) comprising at least a first (14) and a second (18) conveyor on each side of the radiation shielding wall (16) and a device (20) for crossing said radiation shielding wall (16) allowing objects (B) to pass over the radiation shielding wall (16) passing over the top of said radiation shielding wall (16), **characterized in that** the crossing means (20) comprise an arm (22) mounted at a first longitudinal end (22.1) on a shaft (25) capable of rotating about an axis (Y) perpendicular to a displacement direction (X) of the objects (B), said axis (Y) being contained in a plane located between the first (14) and the second (18) conveyor, means (24) of gripping the objects mounted on a second longitudinal end (22.2) of the arm (22), means of rotating the arm such that it pivots between one end (14.1) of the first conveyor (14) and one end (18.1) of the second conveyor (18) and means of actuating the gripping means (24).

2. Sterilization system according to claim 1, in which the routing zone (I) and the evacuation zone (II) each comprises at least one first (14) and one second (18) conveyor on each side of the radiation shielding wall (16) and a device (20) for crossing over said radiation shielding wall (16) so that objects (B) can pass over the radiation shielding wall (16) above said radiation shielding wall (16).

3. Sterilization system according to claim 1 or 2, in which the crossing means advantageously comprise means such that the gripping means move in a succession of horizontal planes.

4. Sterilization system according to claim 3, in which said means may comprise a first fixed pulley (23.1) in which the rotation shaft (25) rotates freely, a second pulley (23.2) mounted free to rotate on the second longitudinal end (22.2) of the arm (22), a belt (23.3) connecting the first (23.1) and second (23.2) pulleys and in which the gripping means (24) are fixed in rotation to the second pulley (23.2).

5. Sterilization system according to one of claims 1 to 4, in which the gripping means (24) include a support (26), two jaws (28, 30), said jaws (28, 30) being hinged free to rotate on the support (26), each about an axis (X1, X2) parallel to the displacement direction (X), elastic return means (36) bringing the jaws (28, 30) back towards each other.

6. Sterilization system according to one of claims 1 to 5, in which the actuation means are not supported by the arm (22).

7. Sterilization system according to claim 6, in which the actuation means comprise an actuation part (38) located at the end (14.1) of the first conveyor (14) and an actuation part (40) located at the end (18.1) of the second conveyor (18).

8. Sterilization system according to claim 7, in which each actuation part comprises a shaft (44) free to rotate perpendicular to the displacement direction (X), said shaft (44) being fitted with two cams (42, 43), each cam (42, 43) being designed to cooperate with each jaw (28, 30) to provoke pivoting of said jaws (28, 30) about their axis of rotation (X1, X2) and means of rotating the shafts (44) by a given angle.

9. Sterilization system according to claim 8, in which each jaw (28, 30) comprises two pins (52, 54, 56, 58) located at each end of said jaw (28, 30), said pins (52, 54, 56, 58) having a parallel axis distinct from the rotation axis of the jaw (28, 30) that supports it, each cam (42, 43) coming into contact with a pin (52, 54, 56, 58) by rotation of the shaft (44).

10. Sterilization system according to claim 8 or 9, in which the means (50) of rotating the shafts (44) by a given angle are formed by rotating pneumatic jacks connected to the shafts (44) by rod transmission systems (50).

11. Sterilization system according to one of claims 1 to 10 in combination with claim 5, in which the objects (B) comprise two ribs (34) and each jaw (28, 30) comprises a groove (35) in which a rib (34) will fit.

12. Sterilization system according to one of claims 1 to 11, comprising means of detecting the position of objects inside each of the zones.

13. Sterilization system according to one of claims 1 to 12, comprising a logic controller controlling the crossing device(s) (20) and treatment of objects by irradiation.

14. Sterilization system according to one of claims 1 to 13, in which the conveyors (9, 14, 18) are chain link or roller belts.
